(19) Europäisches Patentamt
European Patent Office
Office européen des brevets

(11) EP 3 323 848 A1

(12) EUROPEAN PATENT APPLICATION
published in accordance with Art. 153(4) EPC

(43) Date of publication:
23.05.2018 Bulletin 2018/21

(51) Int Cl.:
C08J 7/02 (2006.01)    D06M 15/564 (2006.01)
D06N 3/14 (2006.01)

(21) Application number: 16824191.7

(22) Date of filing: 14.06.2016

(86) International application number:
PCT/JP2016/067644

(87) International publication number:
WO 2017/010211 (19.01.2017 Gazette 2017/03)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 14.07.2015 JP 2015140510

(71) Applicant: DIC Corporation
Tokyo 174-8520 (JP)

(72) Inventors:
• TETSUI Tomohiro
Takaishi-shi
Osaka 592-0001 (JP)
• MAEDA Ryo
Takaishi-shi
Osaka 592-0001 (JP)
• KOMATSUZAKI Kunihiko
Takaishi-shi
Osaka 592-0001 (JP)
• CHIJIWA Hiroyuki
Takaishi-shi
Osaka 592-0001 (JP)
• FUJISHITA Norie
Takaishi-shi
Osaka 592-0001 (JP)

(74) Representative: Adam, Holger
Kraus & Weisert
Patentanwälte PartGmbB
Thomas-Wimmer-Ring 15
80539 München (DE)

(54) COAGULATION MANUFACTURING METHOD

(57) The present invention provides a method for producing a coagulated article, which includes coagulating an aqueous urethane resin composition with an alcohol solvent having 2 to 6 carbon atoms. An object of the present invention is to provide a method for producing a coagulated article, from which a coagulated article having excellent texture can be obtained. By the method of the present invention, a coagulated article having excellent texture can be obtained. Further, an alcohol solvent with the number of carbon atoms being small is used for a coagulating bath, which means that the burden on the environment is small. Therefore, a coagulated article obtained by the method of the present invention can be advantageously used in producing gloves, a coating material, a simulated leather sheet, and the like. With respect to the alcohol solvent, from the viewpoint of achieving a further improvement in the texture of the coagulated article due to having high penetrating property into a resin-blended liquid and an isopropyl group having appropriate hydrophobicity, 2-propanol is preferably used.

FIG. 2

EP 3 323 848 A1

**Description**

Technical Field

The present invention relates to a method for producing a coagulated article which can be used, for example, in producing a simulated leather sheet and the like.

Background Art

An aqueous urethane resin composition having a urethane resin dispersed in an aqueous medium can reduce the burden on the environment, as compared to a conventional organic solvent urethane resin composition, and therefore is recently advantageously used as a material for producing a simulated leather sheet, such as artificial leather or synthetic leather, a coating agent, an adhesive, and the like.

The simulated leather sheet is generally formed from a fibrous substrate, such as nonwoven fabric, and optionally an intermediate layer composed of a porous layer or the like, and a surface layer. With respect to the fibrous substrate, for the purpose of improving the simulated leather sheet in flexing resistance and texture, there is used a fibrous substrate, such as nonwoven fabric, which has been impregnated with an aqueous urethane resin composition and subjected to thermal coagulation (impregnated layer).

As an aqueous urethane resin composition for impregnation of the fibrous substrate, for example, an aqueous urethane resin composition containing a polyurethane resin having a carboxyl group and/or a sulfonic group, a multifunctional quaternary ammonium salt as a heat-sensitive coagulant, and an aqueous medium is disclosed (see, for example, PTL 1).

However, in coagulating the aqueous urethane resin composition by thermal coagulation, the following problems have been pointed out. The urethane resin-blended liquid is once reduced in viscosity by heating, and it is likely that the resin is attached on fiber entanglement points due to capillarity so that the resin binds the fibers. Therefore, the resultant film has poor flexibility and flexural properties so that the film easily suffers breakage.

Citation List

Patent Literature

[PTL 1] JP-A-2015-7172

Summary of Invention

Technical Problem

An object of the present invention is to provide a method for producing a coagulated article, from which a coagulated article having excellent texture can be obtained.

Solution to Problem

In the present invention, there is provided a method for producing a coagulated article, including coagulating an aqueous urethane resin composition with an alcohol solvent having 2 to 6 carbon atoms.

Advantageous Effects of Invention

According to the method of the present invention, a coagulated article having excellent texture can be obtained. Further, an alcohol solvent with the number of carbon atoms being relatively small is used for a coagulating bath, which means that the burden on the environment is small. Therefore, a coagulated article obtained by the method of the present invention can be advantageously used in producing gloves, a coating material, a simulated leather sheet, and the like.

Brief Description of Drawings

[Fig. 1] Fig. 1 is an electron microscope photomicrograph showing a cross-sectional view of the fibrous substrate having a coagulated article obtained in Example 1 (magnification: 200 times).
[Fig. 2] Fig. 2 is an electron microscope photomicrograph showing a cross-sectional view of the fibrous substrate having a coagulated article obtained in Comparative Example 1 (magnification: 200 times).

Description of Embodiments

The method for producing a coagulated article of the present invention includes coagulating an aqueous urethane resin composition with an alcohol solvent having 2 to 6 carbon atoms.

In the present invention, it is essential that an alcohol solvent having 2 to 6 carbon atoms be used as a coagulant. The specific alcohol solvent has appropriately strong hydrophobicity and further has high penetrating property into a resin-blended liquid, and therefore can coagulate the aqueous urethane resin composition, so that a coagulated article having excellent texture can be obtained.

With respect to the alcohol solvent, for example, ethanol, 1-propanol, 2-propanol, 1-butanol, 2-butanol, 2-methyl-2-propanol, 1-pentanol, 2-pentanol, 2-methyl-2-butanol, 1-hexanol, 2-hexanol, cyclohexanol, 2-methyl-2-pentanol, 3-methyl-3-pentanol, or the like can be used. These solvents may be used singly or two or more thereof may be used in combination. Of these, 2-propanol is preferably used from the viewpoint of achieving a further improvement in the texture of the coagulated article because of having high penetrating property into a resin-blended liquid and an isopropyl group having appropriate hydrophobicity.

As specific examples of the method for producing a coagulated article of the present invention, there can be mentioned, for example, a method in which a fibrous substrate is impregnated with an aqueous urethane resin composition, and then the impregnated substrate is further immersed in a coagulating bath of an alcohol solvent having 2 to 6 carbon atoms to produce a coagulated article of the urethane resin; and a method in which a fibrous substrate is immersed in a coagulating bath of an alcohol solvent having 2 to 6 carbon atoms, and then the resultant substrate is further immersed in an aqueous urethane resin composition to produce a coagulated article of the urethane resin. Among these, when the former method is used, a state is formed in which even the inside of the fibrous substrate is filled with the coagulated article of the urethane resin so that the coagulated article is entangled in the fibrous substrate, and therefore the resultant fibrous substrate can be advantageously used as an impregnated layer for a simulated leather sheet. On the other hand, when the latter method is used, a urethane coagulated article layer is formed on the surface and the inside near the surface of the fibrous substrate, and therefore the resultant fibrous substrate can be advantageously used in producing gloves. The impregnated layer for simulated leather sheet is produced by the above-mentioned method, and then a surface layer or a topcoat layer is further formed on the layer by a conventional method, thereby obtaining a simulated leather sheet.

With respect to the fibrous substrate, for example, nonwoven fabric, woven fabric, knitted fabric, or the like can be used. With respect to the material constituting the fibrous substrate, for example, a polyester fiber, a nylon fiber, an acrylic fiber, a polyurethane fiber, an acetate fiber, a rayon fiber, a polylactic acid fiber, cotton, linen, silk, wool, a blended fiber thereof, or the like can be used.

With respect to the method for impregnating the fibrous substrate with the aqueous urethane resin composition, for example, there can be mentioned a method in which the fibrous substrate is placed in a bath containing the aqueous urethane resin composition, and then an extra composition is squeezed out of the substrate using a mangle or the like. The time for the impregnation is, for example, in the range of from 1 to 30 minutes.

Then, the resultant substrate is taken out from the above bath, and further immersed in a coagulating bath containing the alcohol solvent, so that the urethane resin in the aqueous urethane resin composition is coagulated, thereby obtaining the fibrous substrate in a state in which a coagulated article is attached on the surface and the inside of nonwoven fabric. In this case, the time for the immersion and coagulation is, for example, in the range of from 1 to 30 minutes. Further, the coagulating bath upon immersion may be at room temperature, but may be heated to, for example, 30 to 70°C.

With respect to the fibrous substrate having the coagulated article of the urethane resin, if necessary, after the immersion and coagulation, the unnecessary coagulant can be removed by washing by exposing the substrate to running water for, for example, 10 minutes to 2 hours.

With respect to the method for immersing the fibrous substrate in a coagulating bath containing the alcohol solvent, for example, there can be mentioned a method in which the fibrous substrate is directly immersed in a coagulating bath containing the alcohol solvent. The time for the immersion is, for example, in the range of from 5 seconds to 10 minutes.

Then, the resultant substrate is taken out from the coagulating bath, and further immersed in the aqueous urethane resin composition, so that the urethane resin in the aqueous urethane resin composition is coagulated, thereby obtaining the fibrous substrate having a urethane coagulated article layer formed on the surface layer of the fibrous substrate and the inside near the surface layer of the fibrous substrate. In this case, the time for the immersion and coagulation is, for example, in the range of from 1 to 30 minutes. Further, the coagulating bath upon immersion may be at room temperature, but may be heated to, for example, 30 to 70°C.

With respect to the fibrous substrate having the coagulated article of the urethane resin, if necessary, after the immersion and coagulation, the unnecessary coagulant can be removed by washing by exposing the substrate to running water for, for example, 10 minutes to 2 hours.

With respect to the aqueous urethane resin composition usable in the present invention, for example, an aqueous urethane resin composition containing an aqueous urethane resin (A) and an aqueous medium (B) can be used.

The aqueous urethane resin (A) can be, for example, dispersed in the aqueous medium (B) described later, and, for example, an aqueous urethane resin having a hydrophilic group, such as an anionic group, a cationic group, or a nonionic group, an aqueous urethane resin forcibly dispersed in the aqueous medium (B) with an emulsifier, or the like can be used. These aqueous urethane resins (A) may be used singly or two or more thereof may be used in combination. Of these, from the viewpoint of the production stability, an aqueous urethane resin having a hydrophilic group is preferably used, and, from the viewpoint of facilitating coagulation with the coagulant used in the present invention and further performing an improvement in the texture which may be caused by a urethane resin easily filling and being entangled evev in the inside of the fibrous substrate, an aqueous urethane resin having an anionic group is more preferably used.

As a method for obtaining the aqueous urethane resin having an anionic group, for example, there can be mentioned a method in which at least one compound selected from the group consisting of a compound having a carboxyl group and a compound having a sulfonyl group is used as a raw material.

With respect to the compound having a carboxyl group, for example, 2,2'-dimethylolpropionic acid, 2,2'-dimethylolbutanoic acid, 2,2'-dimethylolbutyric acid, 2,2'-valeric acid, or the like can be used. These compounds may be used singly or two or more thereof may be used in combination.

With respect to the compound having a sulfonyl group, for example, 3,4-diaminobutanesulfonic acid, 3,6-diamino-2-toluenesulfonic acid, 2,6-diaminobenzenesulfonic acid, N-(2-aminoethyl)-2-aminoethylsulfonic acid, or the like can be used. These compounds may be used singly or two or more thereof may be used in combination.

Part of or all of the carboxyl groups and sulfonyl groups may be neutralized by a basic compound in the aqueous urethane resin composition. With respect to the basic compound, for example, ammonia, an organic amine, such as triethylamine, pyridine, or morpholine; an alkanolamine, such as monoethanolamine or dimethylethanolamine; a metal base compound containing sodium, potassium, lithium, calcium or the like, or the like can be used.

As a method for obtaining the aqueous urethane resin having a cationic group, for example, there can be mentioned a method in which one or more of compounds having an amino group are used as a raw material.

With respect to the compound having an amino group, for example, a compound having a primary or secondary amino group, such as triethylenetetramine or diethylenetriamine; a compound having a tertiary amino group, e.g., an N-alkyldialkanolamine, such as N-methyldiethanolamine or N-ethyldiethanolamine, or an N-alkyldiaminoalkylamine, such as N-methyldiaminoethylamine or N-ethyldiaminoethylamine, or the like can be used. These compounds may be used singly or two or more thereof may be used in combination.

As a method for obtaining the aqueous urethane resin having a nonionic group, for example, there can be mentioned a method in which one or more of compounds having an oxyethylene structure are used as a raw material.

With respect to the compound having an oxyethylene structure, for example, a polyether polyol having an oxyethylene structure, such as polyoxyethylene glycol, polyoxyethylene polyoxypropylene glycol, or polyoxyethylene polyoxytetramethylene glycol, can be used. These compounds may be used singly or two or more thereof may be used in combination.

With respect to the emulsifier usable in obtaining the aqueous urethane resin forcibly dispersed in the aqueous medium (B), for example, a nonionic emulsifier, such as polyoxyethylene nonyl phenyl ether, polyoxyethylene lauryl ether, polyoxyethylene styryl phenyl ether, polyoxyethylene sorbitol tetraoleate, or a polyoxyethylene-polyoxypropylene copolymer; an anionic emulsifier, such as a fatty acid salt, e.g., sodium oleate, an alkylsulfate salt, an alkylbenzenesulfonic acid salt, an alkylsulfosuccinic acid salt, a naphthalenesulfonic acid salt, a polyoxyethylenealkylsulfuric acid salt, an alkanesulfonate sodium salt, or an alkyldiphenyl ether sulfonate sodium salt; a cationic emulsifier, such as an alkylamine salt, an alkyltrimethylammonium salt, or an alkyldimethylbenzylammonium salt, or the like can be used. These emulsifiers may be used singly or two or more thereof may be used in combination.

With respect to the aqueous urethane resin (A), specifically, an aqueous urethane resin which is obtained from a polyisocyanate (a1), a polyol (a2), raw materials used for producing the above-mentioned aqueous urethane resin having a hydrophilic group, and optionally a chain extender (a3) as raw materials can be used. In the reaction for these materials, a known urethane formation reaction can be utilized.

With respect to the polyisocyanate (a1), for example, an aromatic polyisocyanate, such as phenylene diisocyanate, tolylene diisocyanate, diphenylmethane diisocyanate, xylylene diisocyanate, naphthalene diisocyanate, polymethylene polyphenyl polyisocyanate, or carbodiimidized diphenylmethane polyisocyanate; an aliphatic or alicyclic polyisocyanate, such as hexamethylene diisocyanate, lysine diisocyanate, cyclohexane diisocyanate, isophorone diisocyanate, dicyclohexylmethane diisocyanate, xylylene diisocyanate, tetramethylxylylene diisocyanate, dimer acid diisocyanate, or norbornene diisocyanate, or the like can be used. These polyisocyanates may be used singly or two or more thereof may be used in combination.

With respect to the polyol (a2), for example, polyether polyol, polyester polyol, polyacryl polyol, polycarbonate polyol, polybutadiene polyol, or the like can be used. These polyols may be used singly or two or more thereof may be used in combination.

From the viewpoint of the mechanical strength of the resultant film, the number average molecular weight of the polyol (a2) is preferably in the range of from 500 to 8,000, more preferably in the range of from 800 to 4, 000. The number average molecular weight of the polyol (a2) is a value obtained by the measurement according to a gel permeation chromatography (GPC) method under the conditions shown below.

Measurement apparatus: High-speed GPC apparatus ("HLC-8220GPC", manufactured by Tosoh Corporation)
Columns: The columns shown below, manufactured by Tosoh Corporation, were connected in series for use.

"TSKgel G5000" (7.8 mm I.D. $\times$ 30 cm) $\times$ 1
"TSKgel G4000" (7.8 mm I.D. $\times$ 30 cm) $\times$ 1
"TSKgel G3000" (7.8 mm I.D. $\times$ 30 cm) $\times$ 1
"TSKgel G2000" (7.8 mm I.D. $\times$ 30 cm) $\times$ 1

Detector: RI (differential refractometer)
Column temperature: 40°C
Eluent: Tetrahydrofuran (THF)
Flow rate: 1.0 mL/minute
Sample amount per injection: 100 $\mu$L (tetrahydrofuran solution having a sample concentration of 0.4% by mass)
Standard sample: A calibration curve was prepared using the standard polystyrenes shown below.

(Standard polystyrenes)

"TSKgel standard polystyrene A-500", manufactured by Tosoh Corporation
"TSKgel standard polystyrene A-1000", manufactured by Tosoh Corporation
"TSKgel standard polystyrene A-2500", manufactured by Tosoh Corporation
"TSKgel standard polystyrene A-5000", manufactured by Tosoh Corporation
"TSKgel standard polystyrene F-1", manufactured by Tosoh Corporation
"TSKgel standard polystyrene F-2", manufactured by Tosoh Corporation
"TSKgel standard polystyrene F-4", manufactured by Tosoh Corporation
"TSKgel standard polystyrene F-10", manufactured by Tosoh Corporation
"TSKgel standard polystyrene F-20", manufactured by Tosoh Corporation
"TSKgel standard polystyrene F-40", manufactured by Tosoh Corporation
"TSKgel standard polystyrene F-80", manufactured by Tosoh Corporation
"TSKgel standard polystyrene F-128", manufactured by Tosoh Corporation
"TSKgel standard polystyrene F-288", manufactured by Tosoh Corporation
"TSKgel standard polystyrene F-550", manufactured by Tosoh Corporation

With respect to the chain extender (a3), for example, a chain extender having an amino group, such as ethylenediamine, 1,2-propanediamine, 1,6-hexamethylenediamine, piperazine, 2,5-dimethylpiperazine, isophoronediamine, 1,2-cyclohexanediamine, 1,3-cyclohexanediamine, 1,4-cyclohexanediamine, 4,4'-dicyclohexylmethanediamine, 3,3'-dimethyl-4,4'-dicyclohexylmethanediamine, 1,4-cyclohexanediamine, or hydrazine; a chain extender having a hydroxyl group, such as ethylene glycol, diethylene glycol, triethylene glycol, propylene glycol, dipropylene glycol, 1,3-propanediol, 1,3-butanediol, 1,4-butanediol, hexamethylene glycol, saccharose, methylene glycol, glycerol, sorbitol, bisphenol A, 4,4'-dihydroxydiphenyl, 4,4'-dihydroxydiphenyl ether, or trimethylolpropane, or the like can be used. These chain extenders may be used singly or two or more thereof may be used in combination. Of these, from the viewpoint of achieving a further improvement in the oil grip property and chemical resistance, a chain extender having a hydroxyl group is preferably used.

In the case of using the chain extender (a3), from the viewpoint of achieving a further improvement in the durability of the film, the amount of the chain extender (a3) used is preferably in the range of from 0.5 to 20% by mass, more preferably in the range of from 1 to 10% by mass, based on the total mass of the polyisocyanate (a1), the polyol (a2), and the chain extender (a3).

From the viewpoint of further achieving control of discoloration with time without impairing the texture, the aqueous urethane resin (A) preferably has an urea bond content of 1.2 mol/kg or less.

The urea bond is formed when an amine, which is formed due to a reaction of the chain extender having an amino group or the isocyanate with water, and the polyisocyanate are reacted with each other. Therefore, by controlling the amount of the chain extender having an amino group used and further changing all the isocyanate into urethane prior to the emulsifying operation, it is possible to control the urea bond content of the aqueous urethane resin (A). The urea bond content is a value calculated from the following general formula (1).

$$\text{Urea bond content (mol/kg)} = A/B \qquad (1)$$

wherein A = (Mole of amino group) + (Mole of isocyanate group - Mole of hydroxyl group - Mole of amino group)/2 and

B = Mass of polyol (a2) + Mass of polyisocyanate (a1) + Mass of chain extender (a3)

Further, when an aqueous urethane resin having an aromatic ring obtained using an aromatic polyisocyanate as a raw material is used as the aqueous urethane resin (A), the aromatic ring content of the aqueous urethane resin is preferably in the range of from 0.8 to 8 mol/kg, more preferably in the range of from 1 to 6 mol/kg.

With respect to the method for producing the aqueous urethane resin (A), for example, there can be mentioned a method in which the polyisocyanate (a1) and the polyol (a2) are reacted with each other to produce a urethane prepolymer having an isocyanate group, and then optionally the urethane prepolymer and the chain extender (a3) are reacted with each other to produce an aqueous urethane resin; and a method in which the polyisocyanate (a1), the polyol (a2), and optionally the chain extender (a3) are charged at the same time and reacted with each other. These reactions are conducted, for example, at 50 to 100°C for 3 to 10 hours.

The molar ratio of the isocyanate group of the polyisocyanate (a1) to the total of the hydroxyl group of the polyol (a2) and the hydroxyl group and/or amino group of the chain extender (a3) [(isocyanate group)/(hydroxyl group and/or amino group)] is preferably in the range of from 0.8 to 1.2, more preferably in the range of from 0.9 to 1.1.

In producing the aqueous urethane resin (A), it is preferred that the isocyanate group remaining in the aqueous urethane resin (A) is deactivated. When the isocyanate group is deactivated, an alcohol having one hydroxyl group, such as methanol, is preferably used. The amount of the alcohol used, relative to 100 parts by mass of the aqueous urethane resin (A), is preferably in the range of from 0.001 to 10 parts by mass.

Further, in producing the urethane resin (A), an organic solvent may be used. With respect to the organic solvent, for example, a ketone compound, such as acetone or methyl ethyl ketone; an ether compound, such as tetrahydrofuran or dioxane; an acetate compound, such as ethyl acetate or butyl acetate; a nitrile compound, such as acetonitrile; an amide compound, such as dimethylformamide or N-methylpyrrolidone, or the like can be used. These organic solvents may be used singly or two or more thereof may be used in combination. It is preferred that the organic solvent is removed by a distillation method or the like before obtaining an aqueous urethane resin composition.

With respect to the aqueous medium (B), for example, water, an organic solvent miscible with water, a mixture thereof, or the like can be used. With respect to the organic solvent miscible with water, for example, an alcohol solvent, such as methanol, ethanol, or n- or isopropanol; a ketone solvent, such as acetone or methyl ethyl ketone; a polyalkylene glycol solvent, such as ethylene glycol, diethylene glycol, or propylene glycol; a polyalkylene glycol alkyl ether solvent; a lactam solvent, such as N-methyl-2-pyrrolidone, or the like can be used. These aqueous media may be used singly or two or more thereof may be used in combination. Of these, from the viewpoint of the safety and reduction of the burden on the environment, only water, or a mixture of water and an organic solvent miscible with water is preferably used, and only water is more preferably used.

From the viewpoint of the workability, the mass ratio of the aqueous urethane resin (A) to the aqueous medium (B) [(A)/(B)] is preferably in the range of from 10/80 to 70/30, more preferably in the range of from 20/80 to 60/40.

The aqueous urethane resin composition used in the present invention may optionally contain other additives in addition to the urethane resin (A) and the aqueous medium (B) .

With respect to the other additives, for example, an emulsifier, a neutralizing agent, a thickener, a crosslinking agent, a urethane formation catalyst, a silane coupling agent, a filler, a thixotropic agent, a tackifier, a wax, a heat stabilizer, a light stabilizer, a fluorescent brightener, a foaming agent, a pigment, a dye, an electrical conductivity imparting agent, an antistatic agent, a moisture permeability improver, a water repellent, an oil repellent, a hollow foam, a flame retardant, a water absorbent, a desiccant, a deodorant, a foam stabilizer, an anti-blocking agent, a hydrolysis preventive agent, or the like can be used. These additives may be used singly or two or more thereof may be used in combination.

With respect to the emulsifier, the same emulsifier as those usable in obtaining the aqueous urethane resin forcibly dispersed in the aqueous medium (B) can be used. These emulsifiers may be used singly or two or more thereof may be used in combination. Of these, from the viewpoint of achieving an improvement in the water dispersion stability of the aqueous urethane resin (A) and further improving the texture due to the urethane resin which is likely to fill and be entangled even in the inside of the fibrous substrate, a nonionic emulsifier is preferably used.

In the case of using the emulsifier, from the viewpoint of the water dispersion stability and the texture, the amount of the emulsifier used is preferably in the range of from 0.1 to 30 parts by mass, more preferably in the range of from 1 to 10 parts by mass, relative to 100 parts by mass of the aqueous urethane resin (A).

The neutralizing agent neutralizes the carboxyl group of an anionic aqueous urethane resin which is used as the aqueous urethane resin (A), and, for example, a nonvolatile base, such as sodium hydroxide or potassium hydroxide; a tertiary amine compound, such as trimethylamine, triethylamine, dimethylethanolamine, methyldiethanolamine, or triethanol, or the like can be used. These neutralizing agents may be used singly or two or more thereof may be used in combination.

The amount of the neutralizing agent used is preferably in the range of from 0.8 to 1.2 times the molar number of the carboxyl group contained in the aqueous urethane resin (A).

With respect to the above-described aqueous urethane resin composition used in the present invention, from the viewpoint of further facilitating coagulation with the coagulant, further performing an improvement in the texture, which may be caused by the resin easily filling and being entangled even in the inside of the fibrous substrate, and improving the water dispersion stability, an aqueous urethane resin composition containing an aqueous urethane resin (A) having an anionic group, which is obtained by reacting an aromatic polyisocyanate, a polyol, and a chain extender with one another, an aqueous medium (B), and a nonionic emulsifier, is preferably used.

Examples

Hereinbelow, the present invention will be described in more detail with reference to the following Examples.

[Synthesis Example 1]

Preparation of an aqueous urethane resin composition (X-1)

In the presence of 3,281 parts by mass of methyl ethyl ketone and 0.1 part by mass of tin(II) octylate, 1,000 parts by mass of polycarbonate polyol ("NIPPOLAN 980R", manufactured by Nippon Polyurethane Industry Co., Ltd.; number average molecular weight: 2,000), 17 parts by mass of 2,2'-dimethylolpropionic acid, 47 parts by mass of ethylene glycol, and 344 parts by mass of diphenylmethane diisocyanate were reacted at 70°C until the solution viscosity reached 20, 000 mPa·s, and then 3 parts by mass of methanol was added to terminate the reaction, thereby obtaining a methyl ethyl ketone solution of an aqueous urethane resin (A-1) . Into the obtained urethane resin solution, 70 parts by mass of polyoxyethylene distyrenated phenyl ether (Hydrophile-Lipophile Balance (hereinafter, abbreviated to "HLB") : 14) and 13 parts by mass of triethylamine were mixed, and then 800 parts by mass of ion-exchanged water was added to the resultant mixture to cause phase inversion emulsification, thereby obtaining an emulsion having the aqueous urethane resin (A-1) dispersed in water.

Then, methyl ethyl ketone was distilled off from the emulsion to obtain an aqueous urethane resin composition (X-1) having a nonvolatile content of 40% by mass. The aromatic ring content of the aqueous urethane resin (A-1) was 1.93 mol/kg.

[Synthesis Example 2]

Preparation of an aqueous urethane resin composition (X-2)

In the presence of 3,281 parts by mass of methyl ethyl ketone and 0.1 part by mass of tin(II) octylate, 1,000 parts by mass of polyether polyol ("PTMG2000", manufactured by Mitsubishi Chemical Corporation; number average molecular weight: 2,000), 17 parts by mass of 2,2'-dimethylolpropionic acid, 47 parts by mass of ethylene glycol, and 344 parts by mass of diphenylmethane diisocyanate were reacted at 70°C until the solution viscosity reached 20, 000 mPa·s, and then 3 parts by mass of methanol was added to terminate the reaction, thereby obtaining a methyl ethyl ketone solution of an aqueous urethane resin (A-2). Into the obtained urethane resin solution, 70 parts by mass of polyoxyethylene distyrenated phenyl ether (HLB: 14) and 13 parts by mass of triethylamine were mixed, and then 800 parts by mass of ion-exchanged water was added to the resultant mixture to cause phase inversion emulsification, thereby obtaining an emulsion having the aqueous urethane resin (A-2) dispersed in water.

Then, methyl ethyl ketone was distilled off from the emulsion to obtain an aqueous urethane resin composition (X-2) having a nonvolatile content of 40% by mass. The aromatic ring content of the aqueous urethane resin (A-2) was 1.93 mol/kg.

[Synthesis Example 3]

Preparation of an aqueous urethane resin composition (X-3)

In the presence of 3,281 parts by mass of methyl ethyl ketone and 0.1 part by mass of tin (II) octylate, 1,000 parts by mass of polyester polyol ("Placcel 220", manufactured by Daicel Corporation; number average molecular weight: 2,000), 17 parts by mass of 2,2'-dimethylolpropionic acid, 47 parts by mass of ethylene glycol, and 344 parts by mass of diphenylmethane diisocyanate were reacted at 70°C until the solution viscosity reached 20,000 mPa·s, and then 3 parts by mass of methanol was added to terminate the reaction, thereby obtaining a methyl ethyl ketone solution of an aqueous urethane resin (A-3). Into the obtained urethane resin solution, 70 parts by mass of polyoxyethylene distyrenated phenyl ether (HLB: 14) and 13 parts by mass of triethylamine were mixed, and then 800 parts by mass of ion-exchanged water was added to the resultant mixture to cause phase inversion emulsification, thereby obtaining an emulsion having the aqueous urethane resin (A-3) dispersed in water.

Then, methyl ethyl ketone was distilled off from the emulsion to obtain an aqueous urethane resin composition (X-3) having a nonvolatile content of 40% by mass. The aromatic ring content of the aqueous urethane resin (A-3) was 1.93 mol/kg.

[Synthesis Example 4]

Preparation of an aqueous urethane resin composition (X-4)

In the presence of 1,279 parts by mass of methyl ethyl ketone and 0.1 part by mass of tin(II) octylate, 1,000 parts by mass of polyether polyol ("PTMG2000", manufactured by Mitsubishi Chemical Corporation; number average molecular weight: 2,000), 17 parts by mass of 2,2'-dimethylolpropionic acid, and 262 parts by mass of dicyclohexylmethane diisocyanate were reacted at 70°C until the isocyanate content reached 1.23% by mass, thereby obtaining a methyl ethyl ketone solution of an aqueous urethane resin (A-4). Into the obtained urethane resin solution, 64 parts by mass of polyoxyethylene distyrenated phenyl ether (HLB: 14) and 13 parts by mass of triethylamine were mixed, and then 2,558 parts by mass of ion-exchanged water was added to the resultant mixture to cause phase inversion emulsification, and 607 parts by mass of a 10% by mass solution of isophoronediamine was further mixed into the mixture, thereby obtaining an emulsion having the aqueous urethane resin (A-4) dispersed in water.

Then, methyl ethyl ketone and water were distilled off from the emulsion to obtain an aqueous urethane resin composition (X-4) having a nonvolatile content of 40% by mass.

[Synthesis Example 5]

Preparation of an aqueous urethane resin composition (X-5)

In the presence of 1,279 parts by mass of methyl ethyl ketone and 0.1 part by mass of tin(II) octylate, 1,000 parts by mass of polyester polyol ("Placcel 220", manufactured by Daicel Corporation; number average molecular weight: 2,000), 17 parts by mass of 2,2'-dimethylolpropionic acid, and 262 parts by mass of dicyclohexylmethane diisocyanate were reacted at 70°C until the isocyanate content reached 1.23% by mass, thereby obtaining a methyl ethyl ketone solution of an aqueous urethane resin (A-5). Into the obtained urethane resin solution, 64 parts by mass of polyoxyethylene distyrenated phenyl ether (HLB: 14) and 13 parts by mass of triethylamine were mixed, and then 2, 558 parts by mass of ion-exchanged water was added to the resultant mixture to cause phase inversion emulsification, and 607 parts by mass of a 10% by mass solution of isophoronediamine was further mixed into the mixture, thereby obtaining an emulsion having the aqueous urethane resin (A-5) dispersed in water.

Then, methyl ethyl ketone and water were distilled off from the emulsion to obtain an aqueous urethane resin composition (X-5) having a nonvolatile content of 40% by mass.

[Example 1]

100 Parts by mass of the aqueous urethane resin composition (X-1) obtained in Synthesis Example 1, 5 parts by mass of a thickener ("Borch Gel L75N", manufactured by Borchers), 4 parts by mass of a carbodiimide crosslinking agent ("Carbodilite SV-02", manufactured by Nisshinbo Chemical Inc.), and 200 parts by mass of ion-exchanged water were stirred using a mechanical mixer at 2, 000 rpm for 2 minutes, and then subjected to deaeration using a vacuum deaerator to prepare a blended liquid.

Then, nonwoven fabric (weight per unit area: 250 g/m$^2$) was impregnated with the blended liquid, and then the unnecessary blended liquid was squeezed out of the fabric using a rubber roller mangle so that the impregnation amount became 200%. Then, the nonwoven fabric impregnated with the blended liquid was immersed in a coagulating bath of 2-propanol for 3 minutes to coagulate the blended liquid. Finally, the resultant fabric was dried using a hot air dryer at 100°C for 30 minutes to obtain a fibrous substrate having a coagulated article.

[Examples 2 to 5]

Fibrous substrates having a coagulated article each was obtained in the same manner as in Example 1 except that the aqueous urethane resin composition used was changed as shown in Table 1.

[Comparative Example 1]

100 Parts by mass of the aqueous urethane resin composition (X-1) obtained in Synthesis Example 1 and 100 parts by mass of a 2% by mass aqueous solution of sodium chloride were stirred using a mechanical mixer under conditions at 2, 000 rpm for 2 minutes to prepare an aqueous urethane resin composition for impregnation.

Then, nonwoven fabric (weight per unit area: 250 $g/m^2$) was immersed in a bath containing the above-obtained aqueous urethane resin composition for impregnation, and then the resultant fabric was squeezed using a rubber roller mangle to obtain an immersed fabric impregnated with the urethane resin composition having the same mass as the mass of the nonwoven fabric. Then, the obtained fabric was dried using the Geer-type hot air dryer at 100°C for 10 minutes to obtain a fibrous substrate having a coagulated article formed by thermal coagulation.

[Comparative Example 2]

The impregnation step for a fibrous substrate was performed in the same manner as in Example 1 except that, instead of the coagulating bath of 2-propanol used in Example 1, a coagulating bath of methanol was used. However, the aqueous urethane resin composition (X-1) was not coagulated.

[Comparative Example 3]

The impregnation step for a fibrous substrate was performed in the same manner as in Example 1 except that, instead of the coagulating bath of 2-propanol used in Example 1, a coagulating bath of hexanol was used. However, the aqueous urethane resin composition (X-1) was not coagulated.

[Measurement method for the amount of the aqueous urethane resin attached on a fibrous substrate]

The fibrous substrates having a coagulated article obtained in Examples 1 to 5 and Comparative Examples 1 to 3 each was cut into a 5 cm square, and a mass of each square was measured by means of a precision balance. For comparison, the fibrous substrate before subjected to the impregnation step was cut into a 5 cm square, and a mass of the square was measured by means of a precision balance. A difference between these masses was determined, and an amount of the aqueous urethane resin attached ($g/m^2$) was calculated.

[Evaluation method for the state of the aqueous urethane resin attached on a fibrous substrate]

The fibrous substrates having a coagulated article obtained in Examples 1 to 5 and Comparative Examples 1 to 3 each was observed using Scanning electron microscope "SU3500" (magnification: 200 times), manufactured by Hitachi High-Technologies Corporation, and evaluated in accordance with the followings.

"T": A state in which the aqueous urethane resin is entangled in fibers in the inside of the fibrous substrate is confirmed.
"F": A state in which the aqueous urethane resin is entangled in fibers in the inside of the fibrous substrate is not confirmed.

[Evaluation method for the texture]

With respect to the fibrous substrates having a coagulated article obtained in Examples 1 to 5 and Comparative Examples 1 to 3, the feel of touch by hand was evaluated in accordance with the followings.

"A": Excellent in tension and stiffness feeling as well as solid feeling.
"B": Tension and stiffness feeling and solid feeling are felt.
"C": Slightly poor in tension and stiffness feeling and solid feeling.

"D": Neither tension and stiffness feeling nor solid feeling are felt at all.

[Example 6]

100 Parts by mass of the aqueous urethane resin composition (X-1) obtained in Synthesis Example 1 and 5 parts by mass of 5% by mass carboxymethyl cellulose diluted with water were stirred using a mechanical mixer at 2, 000 rpm for 2 minutes, and then subjected to deaeration using a vacuum deaerator to obtain a blended liquid.

Then, a knitted glove made of a nylon fiber was set on a glove mold, and the mold was immersed in 2-propanol for 15 seconds, and then further immersed in the above-obtained blended liquid for 10 seconds and pulled up from the blended liquid. Then, the resultant glove mold was dried using a hot air dryer at 70°C for 20 minutes and at 120°C for 20 minutes, and the knitted glove was taken out from the mold, thereby obtaining a glove having a coagulated film.

[Examples 7 to 10]

Gloves having a coagulated film each was obtained in the same manner as in Example 1 except that the aqueous urethane resin composition used was changed as shown in Table 2.

[Comparative Example 4]

100 Parts by mass of the aqueous urethane resin composition (X-1) obtained in Synthesis Example 1, 1 part by mass of sodium chloride, and 5 parts by mass of 5% by mass carboxymethyl cellulose diluted with water were stirred using a mechanical mixer at 2,000 rpm for 2 minutes, and then subjected to deaeration using a centrifugal deaerator to obtain a blended liquid.

Then, a knitted glove made of a nylon fiber was set on a glove mold, and the mold was immersed in the above-obtained blended liquid for 10 seconds and pulled up from the blended liquid. Then, the resultant glove mold was dried using a hot air dryer at 70°C for 20 minutes and at 120°C for 20 minutes, and the knitted glove was taken out from the mold, thereby obtaining a glove having a coagulated film.

[Comparative Example 5]

The immersion step for a fibrous substrate was performed in the same manner as in Example 1 except that, instead of the coagulating bath of 2-propanol used in Example 6, a coagulating bath of methanol was used. However, the aqueous urethane resin composition (X-1) was not coagulated.

[Comparative Example 6]

The immersion step for a fibrous substrate was performed in the same manner as in Example 1 except that, instead of the coagulating bath of 2-propanol used in Example 6, a coagulating bath of hexanol was used. However, the aqueous urethane resin composition (X-1) was not coagulated.

[Evaluation method for formation of the coagulated aqueous urethane resin on the surface of gloves]

The gloves obtained in Examples 6 to 10 and Comparative Examples 4 to 6 each was visually observed, and evaluated in accordance with the followings.

"T": A coagulated film is formed.
"F": No coagulated film is formed.

[Evaluation method for the texture of gloves]

With respect to the gloves obtained in Examples 6 to 10 and Comparative Examples 4 to 6, the feel of touch by hand was evaluated in accordance with the followings.

"A": Excellent in flexibility.
"B": Slightly excellent in flexibility.
"C": Not flexible.

Table 1

| | Example 1 | Example 2 | Example 3 | Example 4 | Example 5 | Comparative Example 1 | Comparative Example 2 | Comparative Example 3 |
|---|---|---|---|---|---|---|---|---|
| Aqueous urethane resin composition | (X-1) | (X-2) | (X-3) | (X-4) | (X-5) | (X-1) | (X-1) | (X-1) |
| Aqueous urethane resin (A) | (A-1) | (A-2) | (A-3) | (A-4) | (A-5) | (A-1) | (A-1) | (A-1) |
| Coagulation method | IPA | IPA | IPA | IPA | IPA | Thermal coagulation | Methanol | Hexanol |
| Processing method (1) | | | | | | | | |
| Amount of aqueous urethane resin attached on fibrous substrate (g/m$^2$) | 70 | 69 | 70 | 68 | 69 | 71 | | |
| Evaluation of state of aqueous urethane resin attached on fibrous substrate | T | T | T | T | T | F | Not coagulated | Not coagulated |
| Evaluation of texture | A | A | A | A | A | D | | |

| Table 2 | Example 6 | Example 7 | Example 8 | Example 9 | Example 10 | Comparative Example 4 | Comparative Example 5 | Comparative Example 6 |
|---|---|---|---|---|---|---|---|---|
| Aqueous urethane resin composition | (X-1) | (X-2) | (X-3) | (X-4) | (X-5) | (X-1) | (X-1) | (X-1) |
| Aqueous urethane resin (A) | (A-1) | (A-2) | (A-3) | (A-4) | (A-5) | (A-1) | (A-1) | (A-1) |
| Coagulation method | IPA | IPA | IPA | IPA | IPA | Thermal coagulation | Methanol | Hexanol |
| Processing method (2) | | | | | | | | |
| Formation of coagulated aqueous urethane resin on surface of gloves | T | T | T | T | T | T | Not coagulated | Not coagulated |
| Evaluation of texture of gloves | A | A | A | A | A | C | | |

The abbreviation shown in Tables 1 and 2 is as follows. "IPA": 2-Propanol

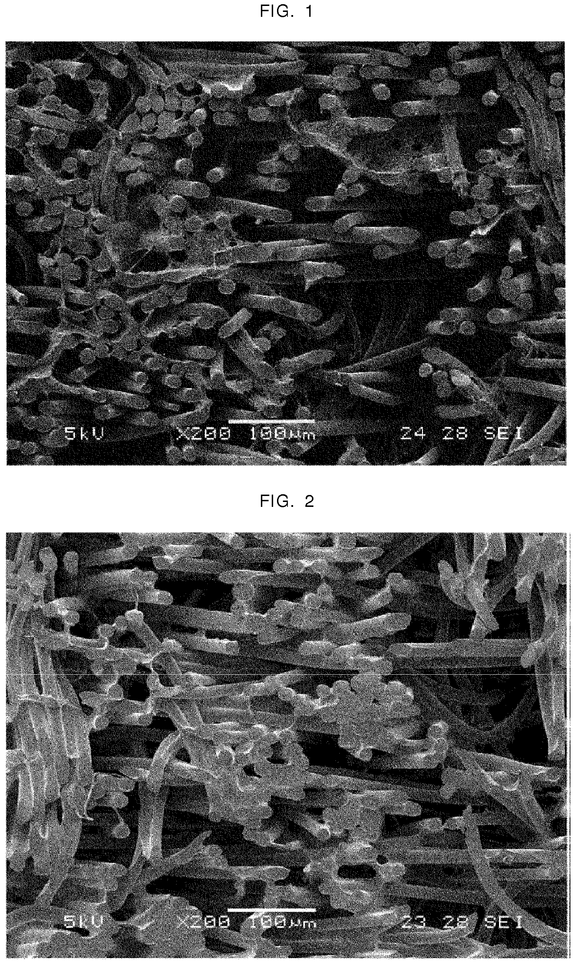

With respect to Examples 1 to 3 which correspond to the present invention, it has been found that a coagulated article having excellent texture can be obtained. Further, as can be seen from Fig. 1, it has been found that, by employing a specific method for producing a coagulated article, a state is formed in which even the inside of the fibrous substrate is filled with the coagulated article of the urethane resin so that the coagulated article is entangled in the fibrous substrate. Further, in all Examples 4 to 6 which are an example in which gloves were produced using the method of the present invention, the texture was excellent.

On the other hand, in Comparative Example 1 which is an embodiment in which coagulation was conducted by thermal coagulation, the texture was poor. Further, as can be seen from Fig. 2, the inside of the fibrous substrate was not filled with the urethane resin, and no entanglement of the urethane resin in the fibrous substrate was confirmed. Further, in Comparative Example 4 which is an example in which gloves were produced by thermal coagulation, the texture was poor.

In all Comparative Examples 2, 3, 5, and 6 which are an embodiment in which, instead of 2-propanol, the other coagulating bath was used, no coagulation was caused.

**Claims**

1. A method for producing a coagulated article, comprising: coagulating an aqueous urethane resin composition with an alcohol solvent having 2 to 6 carbon atoms.

2. The method for producing a coagulated article according to claim 1, comprising the step of impregnating a fibrous substrate with the aqueous urethane resin composition, and then immersing the resultant fibrous substrate in a coagulating bath of the alcohol solvent having 2 to 6 carbon atoms.

3. The method for producing a coagulated article according to claim 1, comprising the step of immersing a fibrous substrate in a coagulating bath of the alcohol solvent having 2 to 6 carbon atoms, and then immersing the resultant fibrous substrate in the aqueous urethane resin composition.

4. The method for producing a coagulated article according to any one of claims 1 to 3, wherein the alcohol solvent is 2-propanol.

FIG. 1

FIG. 2

## INTERNATIONAL SEARCH REPORT

| International application No. |
| --- |
| PCT/JP2016/067644 |

### A. CLASSIFICATION OF SUBJECT MATTER

*C08J7/02*(2006.01)i, *D06M15/564*(2006.01)i, *D06N3/14*(2006.01)i

According to International Patent Classification (IPC) or to both national classification and IPC

### B. FIELDS SEARCHED

Minimum documentation searched (classification system followed by classification symbols)
B29C71/04, C08J7/02, D06M13/00-15/715, D06N1/00-7/06

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/REGISTRY(STN)

### C. DOCUMENTS CONSIDERED TO BE RELEVANT

| Category* | Citation of document, with indication, where appropriate, of the relevant passages | Relevant to claim No. |
| --- | --- | --- |
| X <br> Y | Osamu FUKUSHIMA, Yoshitami SAITO, "Chemical Structure and Coagulation of Polyurethane", Japanese Journal of Polymer Science and Technology, 1982.09, vol.39, no.9, pages 535 to 542, particularly, summary, tables 1, 2 | 1 <br> 1-2,4 |
| X <br> Y | JP 53-49194 A (Toyo Cloth Co., Ltd.), 04 May 1978 (04.05.1978), claims; page 2, lower right column, lines 11 to 19 <br> (Family: none) | 1-2,4 <br> 1-2,4 |
| X | Q. XING et al., Morphology and performance control of PLLA-based porous membranes by phase separation, Polymer, 2013.08.11, vol.54, pp.5965-5973, particularly, p.5967, 2.1, 2.2, Table 1 | 1 |

| ☒ Further documents are listed in the continuation of Box C. | ☐ See patent family annex. |
| --- | --- |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search <br> 05 September 2016 (05.09.16) | Date of mailing of the international search report <br> 13 September 2016 (13.09.16) |
| --- | --- |
| Name and mailing address of the ISA/ <br> Japan Patent Office <br> 3-4-3,Kasumigaseki,Chiyoda-ku, <br> Tokyo 100-8915,Japan | Authorized officer <br><br> Telephone No. |

Form PCT/ISA/210 (second sheet) (January 2015)

**INTERNATIONAL SEARCH REPORT**

International application No.

PCT/JP2016/067644

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y | J.S. SADOWSKI et al., Polyurethane Latexes for Coagulation Dipping, Elastomerics, 1978.08, vol.110, No.8, pp.17-20, particularly, page 18, left column, lines 16 to 24, page 18, right column, column 3, lines 26 to 34 | 1-2<br>3 |
| X<br>Y | WO 2015/012117 A1  (DIC Corp.), 29 January 2015 (29.01.2015), claims; paragraphs [0056], [0060] & TW 201503842 A | 1,3-4<br>3 |
| Y | WO 2009/119551 A1  (Kuraray Co., Ltd.), 01 October 2009 (01.10.2009), paragraph [0094] & US 2011/0020590 A1 paragraph [0101] & EP 2261382 A1          & CN 102016077 A & KR 10-2010-0130221 A | 2 |
| Y | JP 2007-224471 A  (Dainippon Ink and Chemicals, Inc.), 06 September 2007 (06.09.2007), paragraph [0040] (Family: none) | 2 |
| A | L.-P. CHENG et al., Effect of the temperature of polyurethane dissolution on the mechanism of wet-casting membrane formation, European Polymer Journal, 2003, vol.39, pp.601-607 | 1-4 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015007172 A